# EUROPÄISCHE PATENTSCHRIFT

(11) **EP 0 563 635 B1**
(45) Veröffentlichungstag und Bekanntmachung des Hinweises auf die Patenterteilung: **19.08.1998**
(21) Anmeldenummer: 93103822.8
(22) Anmeldetag: 10.03.1993
(51) Int. Cl.: G01N 33/574, G01N 33/58

(54) **Verfahren zum Nachweis von Mikrometastasen in mesenchymalem Gewebe**
Method for detecting micrometastases in mesenchymal tissue
Méthode pour la détection de micrometastases dans les tissus mésenchymales

(30) Priorität: 16.03.1992 DE 4208422
(43) Veröffentlichungstag der Anmeldung: 06.10.1993
(73) Patentinhaber: BOEHRINGER MANNHEIM GMBH, 68298 Mannheim (DE)
(72) Erfinder: Dr. Heinz Bodenmüller, D-8132 Tutzing (DE); Prof. Dr. Gert Riethmüller, D-8000 München 40 (DE)

(56) Entgegenhaltungen:
- EP-A- 0 267 355
- EP-A- 0 449 269
- WO-A-91/10139
- WO-A-92/02558
- EUR. J. CANCER Bd. 27, Nr. 11, 1991, Seiten 1461 - 1465 G. SCHLIMOK 'Micrometatastatic tumour cells in bone marrow of patients with gastric cancer: methodological aspects of detection an prognostic significance.'
- DATABASE WPI Section Ch, Week 9217, Derwent Publications Ltd., London, GB; Class B04, AN 92-139227 [17] & SU-A-1 649 444 (DON MED INST)
- H. U. BERGMEYER 'Methods of enzymatic analysis, Band II, 3. Auflage' 1983 , VERLAG CHEMIE , WEINHEIM
- "Molecular Cell Biology", DARNELL James E., Scientific American Books, New York, USA, 1986, page 847

## Beschreibung

Die Erfindung betrifft ein Verfahren zum Nachweis von Mikrometastasen ekto- und entodermaler Tumoren in mesenchymalem Gewebe mittels fraktionierter Zellyse in Kombination mit einem Sandwich-Immunoassay.

Für die Prognose eines Tumorleidens ist der Nachweis von Mikrometastasen von großer Bedeutung. Eine systemische Dissemination der Tumorzellen tritt oft schon vor der Diagnose oder Entfernung des Primärtumores auf. Gelingt ein frühzeitiger Nachweis der Metastasierung, so kann diese oft erfolgversprechend zum Beispiel durch eine Chemotherapie behandelt werden. Andererseits lassen sich nach der operativen Entfernung des Primärtumores überflüssige Nachbehandlungen vermeiden, falls sichergestellt werden kann, daß keine Metastasen vorhanden sind.

Bei epithelialen Tumoren ekto- oder entodermalen Ursprungs ist der Nachweis von Mikrometastasen durch einen erhöhten Serumspiegel von tumorspezifischen Antigenen, wie zum Beispiel CEA (Carcinoembryonales Antigen), CA 15-3, CA 19-9, CA 125, PSA (Prostata spezifisches Antigen) oder Cytokeratinfragmenten, sehr problematisch, da auch bei verschiedenen Entzündungen aus normalen nicht entarteten Epithelien solche Antigene ins Serum abgegeben werden. Aus Änderungen der Konzentration dieser Marker für epitheliale Tumoren kann daher zwar auf das Vorhandensein von fortgeschrittenen Tumoren wie beispielsweise in der WO 91/10139 beschrieben, nicht jedoch auf das Vorhandensein von Mikrometastasen geschlossen werden.

Von Schlimok et al., Eur. J. Cancer, Vol.27, 1461-1465, 1991 wird eine Methode zum Nachweis von Mikrometastasen im Knochenmark von Tumorpatienten beschrieben. Mittels einer relativ zeitaufwendigen immuncytologischen Methode können einzelne epitheliale Tumorzellen oder kleine Zellanhäufungen epithelialen Ursprungs im Knochenmark nachgewiesen werden. Knochenmark wurde mittels Punktion entnommen und die mononukleären Zellen durch Dichtegradientenzentrifugation, anschließendes Waschen und erneute Zentrifugation aufbereitet. Die Zellen wurden auf Objektträger zentrifugiert und fixiert. In einem nächsten Schritt wurden monoklonale Antikörper gegen spezifische epitheliale Antigene bevorzugt Cytokeratine aufgebracht. Die Antikörperreaktion wurde durch die APAAP-Technologie (Cordell et al., J. Histochem. Cytochem. 32, 219-229, 1984) mittels eines polyvalenten Kaninchen-Antiserums gegen Maus-Ig und vorgebildeten Komplexen aus alkalischer Phosphatase und monoklonalen Antikörper gegen alkalische Phosphatase nachgewiesen. Positive Zellen zeigten eine cytoplasmatische Verfärbung und konnten ohne Gegenfärbung mikroskopisch ausgewertet werden. Diese Methode kann auch mit enzymmarkierten, fluoreszenzmarkierten oder radioaktiv markierten Antikörpern durchgeführt werden. Für ein Routineverfahren zur Untersuchung einer Vielzahl von Patienten im klinischen Laborbetrieb erscheint dieses Verfahren zu aufwendig.

Aufgabe der vorliegenden Erfindung war daher ein einfaches Verfahren zum Nachweis von Mikrometastasen ekto- oder entodermalen Tumoren bereitzustellen.

Gelöst wird diese Aufgabe durch die in den Ansprüchen näher charakterisierte Erfindung. Insbesondere wird die Aufgabe gelöst durch ein Verfahren zum Nachweis von Mikrometastasen ektound entodermaler Tumoren, dadurch gekennzeichnet, daß Zellen aus mesenchymatischem Gewebe lysiert und im Zellüberstand spezifische ekto- oder entodermale Antigene immunologisch nachgewiesen werden, ohne daß auf den Zellüberstand abbauend eingewirkt wird.

Ein weiterer Gegenstand der Erfindung ist die Verwendung eines Testkits zum Nachweis von Mikrometastasen, das die zur Zellyse und zum immunologischen Nachweis notwendigen Reagenzien in mindestens zwei getrennten Verpackungseinheiten enthält.

Überraschenderweise wurde festgestellt, daß durch das erfindungsgemäße Verfahren bereits wenige Zellen ekto- oder entodermalen Ursprungs in mesenchymalem Gewebe, wie beispielsweise Knochenmark oder Blutzellen, in dem normalerweise diese Zellen nicht vorkommen, nachgewiesen werden können. Es ist somit möglich schon das Anfangsstadium einer Metastasierung von ektooder entodermalen Tumoren zu erkennen. Bei diesen Tumoren handelt es sich meist um epitheliale Tumore, die ekto- oder entodermalen Ursprungs sein können. Hierunter fallen beispielsweise Karzinome wie das weitverbreitete Mammakarzinom, das Magenkarzinom, das colorektale Karzinom, Tumoren des Urogenitaltraktes beispielsweise Blasen-, Nieren- oder Prostatakarzinome oder das nicht kleinzellige Bronchialkarzinom. Als Tumormarker können prinzipiell alle Marker dienen, die nicht in mesenchymalem Gewebe vorkommen, z.B. Cytokeratine, Mucine, Hormonrezeptoren wie EGF- oder Östrogenrezeptoren, Proteasen wie Cathepsine, Tumormarker wie CEA, CA 15-3, PSA (Prostata spezifisches Antigen), CA 19-9, CA 72-4 oder Onkogene wie myc, p53, ras und HER-2. Besonders bevorzugt werden als spezifische Antigene Cytokeratine oder Cytokeratinfragmente genutzt. Diese sind als Intermediär-Filamentproteine Bestandteile des Zytoskeletts von Epithelzellen. Es sind 20 verschiedene Cytokeratine bekannt, von denen die Cytokeratine 1 bis 8 als basische und die Cytokeratine 9 bis 20 als saure Cytokeratine bezeichnet werden. Die Komplexität und Zusammensetzung der Cytokeratine ist unterschiedlich in den verschiedenen epithelialen Geweben, d. h. Epithelzellen haben eine für das jeweilige Gewebe typische Cytokeratin-Zusammensetzung. Daher ist es zum Teil möglich aus den jeweils im mesenchymalen Gewebe nachgewiesenen Cytokeratinen Rückschlüsse auf den Primärtumor zu ziehen.

Intakte Cytokeratin-Moleküle sind als integrale Bestandteile der Intermediär-Filamente der Zellen schwer wasserlöslich. Daher war es besonders überraschend, daß nach einer normalen Zellyse ohne jegliche weitere Vorbehandlung des Zellüberstandes diese Cytokeratine oder Fragmente hiervon durch einen einfachen immunologischen Nachweis bestimmt werden konnten. In der EP-B-0 267 355 wird ausdrücklich beim immunologischen Nachweis von Intermediärfilamentproteinen ein zusätzlicher Arbeitsschritt zum Löslichmachen der Proteine verlangt. Die Gewebeprobe wird bei diesem Verfahren einer enzymatischen Verdauung unterzogen.

Bei dem erfindungsgemäßen Verfahren wird mesenchymales Gewebe entnommen. Bevorzugt wird Knochenmark genutzt. Dieses wird mit einer üblichen Punktionsnadel entnommen. Es empfiehlt sich eine doppelte Punktion, um die Trefferquote zu erhöhen.

Es hat sich als vorteilhaft erwiesen, vor der eigentlichen Lyse der mesenchymatischen Zellen die entnommene Gewebeprobe aufzureinigen. Hauptsächlich sollen hierdurch die Erythrozyten von den restlichen kernhaltigen Zellen abgetrennt werden. Hierzu kann beispielsweise eine Dichtegradientenzentrifugation vorgenommen werden. Die kernhaltigen Zellen können aber auch beispielsweise durch magnetische Partikel, die spezielle Rezeptoren für die kernhaltigen Zellen tragen, gebunden und anschließend in üblicher Weise abgetrennt werden. Am geeignetsten hat sich eine Lyse der Erythrozyten erwiesen. Das mesenchymatische Gewebe wird dabei einer fraktionierten Zellyse unterzogen. Hierzu werden die Erythrozyten mit einen Lysepuffer behandelt. Bei diesem Puffer handelt es sich bevorzugt um einen Ammoniumchlorid-haltigen Puffer. Ammoniumchlorid ist bevorzugt in Konzentrationen von 80 bis 100 g/l enthalten. Der pH-Wert beträgt 7,0 bis 8,0. Es können noch weitere Hilfsstoffe wie zum Beispiel Komplexbildner wie EDTA enthalten sein.

Die mesenchymatischen Zellen werden mit einer 10 - 100fachen Menge des Lysepuffers vermischt und einige Minuten, im allgemeinen reichen 5 Minuten, bevorzugt bei Raumtemperatur inkubiert. Anschließend werden die verbliebenen nicht lysierten kernhaltigen Zellen abzentrifugiert. Das Zellpellet wird in Puffer, bevorzugt PBS-Puffer resuspendiert. Falls gewünscht kann an dieser Stelle die Zellzahl ermittelt und auf einen bestimmten Wert eingestellt werden. Dies erlaubt einen genaueren Vergleich unterschiedlicher Proben. Die kernhaltigen Zellen werden nach erneuter Zentrifugation und Resuspension bevorzugt in gepufferter physiologischer Kochsalzlösung lysiert.

Die Lyse kann durch eine Behandlung mit einem Detergens, zum Beispiel Nonidet P40, wobei eine 30 minütige Inkubation bei Raumtemperatur ausreicht, durch Einfrieren bei -20°C oder aber bevorzugt durch eine Ultraschallbehandlung erfolgen. Nach erneuter Zentrifugation zur Abtrennung der unlöslichen Zelltrümmer werden im überstand die spezifischen Antigene der ektooder entodermalen Zellen immunologisch nachgewiesen.

Zum immunologischen Nachweis können alle üblichen immunologischen Verfahren angewendet werden, die eine ausreichende Spezifität und Sensitivität gewährleisten. Bevorzugt sind dies vor allem heterogene Verfahren. Am geeignetsten sind Sandwich-Assays, bei denen die Probe mit mindestens zwei Rezeptoren R1 und R2 inkubiert wird, die mit dem nachzuweisenden spezifischen Antigen spezifisch bindefähig sind. Der Rezeptor R1 ist dabei an eine Festphase gekoppelt oder kann im Laufe des Testverfahrens an diese gekoppelt werden. Methoden hierzu sind dem Fachmann bekannt, beispielsweise die Kopplung über das Bindungspaar Biotin/Avidin bzw. Streptavidin. Der Rezeptor R2 enthält eine Markierung, beispielsweise ein Enzym, eine fluoreszierende, chemielumineszierende oder radioaktive Gruppe.

Als Rezeptoren werden bevorzugt Antikörper, besonders bevorzugt monoklonale Antikörper gegen die tumorspezifischen Antigene eingesetzt. Um eine hohe Spezifität des immunologischen Testes zu gewährleisten sollten die Rezeptoren möglichst an unterschiedliche Epitope des Antigens binden. Beispiele solcher Rezeptorenpaare sind für verschiedene Tumorantigene bekannt (Int. J. Cancer, 38,47-53 (1986); T. A. Waldmann, Science, 252,1657 ff (1991); Int. J. Cancer, 3,50-55 (1988)). Für den Nachweis von Cytokeratin 19, das für den Nachweis von epithelialen Tumoren besonders geeignet erscheint, wird in der WO 91/10139 ein solches Rezeptorenpaar beschrieben. Das hierin beschriebene Verfahren ist bei dem erfindungsgemäßen Verfahren zum Nachweis von Mikrometastasen im Knochenmark geeignet.
Die Empfindlichkeit der immunologischen Nachweismethode sollte so hoch liegen, daß weniger als 1000 Zellen, bevorzugt weniger als 100 Zellen ekto- oder entodermalen Ursprungs unter 5 x 10⁶ Knochenmarksleukozyten nachgewiesen werden können. Diese Sensitivität wird beispielsweise in dem in der WO 91/10139 beschriebenen Enzym-Immunoassay erreicht.

Falls die Notwendigkeit besteht, die Sensitivität eines immunologischen Tests zu erhöhen, wenn beispielsweise die spezifischen Antigene nur in sehr geringer Konzentration vorkommen, ist dies möglich indem ein Rezeptorengemisch eingesetzt wird.

An der Festphasenseite kann beispielsweise ein Gemisch aus zwei oder mehr Rezeptoren (R1a, R1b, etc), bevorzugt monoklonale Antikörper, eingesetzt werden. Die Rezeptoren R1a, R1b, etc. sind gegen verschiedene spezifische ekto- oder entodermale Antigene gerichtet. Im Falle der Cytokeratine können beispielsweise Antikörper gegen Cytokeratin 8, 18 und 19 gleichzeitig eingesetzt werden oder ein Antikörper, der mehrere Cytokeratine spezifisch erkennt, beispielsweise ein monoklonaler Antikörper, der konservative Epitope der Cytokeratine erkennt. Da bei einem Vorkommen von Mikrometastasen zu erwarten ist, daß die Konzentration der spezifischen Antigene im Zellüberstand sehr niedrig liegt und die Bindekapazität der Festphase durch ein einzelnes Antigen nicht überschritten wird, wird bei Anwesenheit von mehreren verschiedenen spezifischen Rezeptoren gegen verschiedene Antigene insgesamt mehr Antigenmaterial gebunden im Vergleich zum Vorhandensein von einem spezifischen Rezeptor.

In dem Fall, daß mehrere spezifische Antigene an die Festphase gekoppelt werden, muß als Rezeptor R2, der die Markierung trägt, ebenfalls ein Gemisch aus Rezeptoren R2a, R2b, etc. eingesetzt werden, die jeweils ein bestimmtes Antigen zu binden vermögen, bevorzugt an anderen Epitopen wie die Rezeptoren Rla, Rlb, etc. Auch hier kann als R2 ein Antikörper eingesetzt werden, der alle zu bestimmenden Antigene gleichzeitig erkennt, falls die Antigene identische oder sehr nahe verwandte Epitope besitzen.

Bevorzugt wird als Festphase bei dem immunologischen Nachweis eine Mikrotiterplatte verwendet, die mit dem Rezeptor R1 beschichtet ist, eingesetzt, um in einem Testansatz verschiedene Verdünnungsstufen des Probematerials gleichzeitig testen zu können. Falls der Rezeptor R1 nicht direkt an der Festphase gebunden ist, sondern erst im Verlaufe des Tests über ein Bindepaar wie Biotin/Streptavidin gebunden werden soll, ist an der Festphase ein Partner des Bindepaares immobilisiert, im Falle von Biotin/Streptavidin bevorzugt Streptavidin.

Das erfindungsgemäße Verfahren wird durch die folgenden Beispiele näher erläutert:

### Beispiel 1: Nachweis von Mikrometastasen epithelialer Tumoren im Knochenmark mittels eines Cytokeratin-ELISA

### Knochenmarksaufbereitung durch fraktionierte Lyse

Knochenmark wird mit einer Punktionsnadel aus der Cristae iliacae posteriores des Beckenkammes präoperativ entnommen. Pro Punktionsstelle werden 3 - 6 ml Knochenmark in heparinisierten Spritzen entnommen. Das Knochenmark wird bei 4°C gelagert. Zur Lyse der Erythozyten wird 1 ml Knochenmark in 40 ml Lysepuffer 5 Minuten bei Raumtemperatur inkubiert. Der Lysepuffer hat die folgende Zusammensetzung:
89,9 g/l Ammoniumchlorid
10,0 g/1 KHCO₂
370 mg/l EDTA

Der Lysepuffer wird vor der Benutzung sterilfiltriert.

Nach der Inkubation wird 5 Minuten bei 300g und Raumtemperatur zentrifugiert. Der Überstand wird verworfen und das Zellpellet in 1 ml PBS resuspendiert. In dieser Suspension wurde die Zellzahl bestimmt, die Zellsuspension aliquotiert und auf 5 x 10⁶ Zellen/Aliquot eingestellt. Nach einer erneuten Zentrifugation bei 8000 g für 3 Minuten in einer Eppendorff-Zentrifuge wird der Überstand abpipettiert und die Zellen im Pellet in 30 µl 0,9% NaCl-Lösung resuspendiert. Die kernhaltigen Zellen wurden durch eine 10minütige Ultraschallbehandlung lysiert und 220 µl 40 mM Phosphat-Puffer pH 7,4 hinzugefügt. Nach erneuter Zentrifugation zur Abtrennung der Zellfragmente wird der Überstand im Cytokeratin-19-ELISA eingesetzt.

### Cytokeratin-19-ELISA

Der Cytokeratin-19-ELISA wurde ähnlich wie in der WO 91/10139 beschrieben durchgeführt. Es wurden die gleichen Reagenzien und monoklonalen Antikörper verwendet. Die Empfindlichkeit des Testes konnte durch eine schrittweise Testführung und durch zufügen von 12 g/l Polyethylenglykol, mit einem Molekulargewicht von 40000, im Puffer gegenüber dem in der WO 91/10139 beschriebenen Test noch erhöht werden. In einer Mikrotiterplatte, die mit Streptavidin beschichtet ist, werden 100 µl einer Lösung eines biotiylierten Antikörpers Ks 19.1 in 40 mM PEG-haltigem Phosphat-Puffer pH 7,4 pipettiert und 60 Minuten inkubiert. Nach dreimaligem Waschen wird 100 µl Knochenmarksextrakt, der wie zuvor beschrieben aufbereitet wurde, hinzupipettiert und 90 Minuten bei Raumtemperatur inkubiert. Anschließend wird erneut dreimal gewaschen. Nach Zugabe 100 µl einer Lösung eines Peroxidase-markierten monoklonalen Antikörpers BM 19 in 40 mM PEG-haltigem Phosphat-Puffer pH 7,4 wird erneut 90 Minuten inkubiert. Nach dreimaligem Waschen wird mit 100 µl der Enzymsubstratlösung ABTS® (100 mM Phosphat-Citrat-Puffer pH 5,0, 1,47 mM Natriumperborat, 9,1 mM ABTS®) bei Raumtemperatur inkubiert und nach 60 Minuten die Extinktion bei 405 nm als Maß für die Analytkonzentration gemessen.

Im Modellversuch erreichte der Test eine Nachweisempfindlichkeit bis zu 10 HT-29 Zellen (Zellen einer Kolonkarzinomzelllinie) in 5 x 10⁶ Leukozyten. HT-29-Zellen wurden in steigender Konzentration zu 1 ml Vollblut zugemischt. Anschließend wurde entsprechend dem oben beschriebenen Protokoll lysiert und die Cytokeratinkonzentration bestimmt.

Die Knochenmarksproben von 99 Karzinompatienten wurden parallel mit dem Cytokeratin-19-ELISA und mit Hilfe der Immuncytologie (Schlimok et al., Eur. J. Cancer 27, 1461-1465, 1991) untersucht. Die Befunde von 64 (65%) stimmten in beiden Verfahren überein, 41 Patienten hiervon waren negativ, 23 Patienten positiv. Bei 13 weiteren Patienten mit negativem Cytologieergebnis wurden im Cytokeratin-19-ELISA ein erhöhter Cytokeratingehalt, d.h. mehr als 100 pg, nachgewiesen. Lediglich 22 Patienten mit positivem Cytologiebefund waren im Cytokeratin-19-ELISA negativ. Bei Anwendung des Chiquadrattestes ergab sich eine signifikante Übereinstimmung (X² = 7,75, p > 0,01) der Nachweismethoden.

Bei 34 der 99 Patienten wurde an zwei Punktionsstellen Knochenmark entnommen. Hier stimmten beide Verfahren bezogen auf die Punktionsstellen (n = 133) sogar in 70% der Befunde überein.

## Patentansprüche

1. Verfahren zum Nachweis von Mikrometastasen ekto- oder entodermaler Tumoren, dadurch gekennzeichnet, daß Zellen aus mesenchymatischem Gewebe lysiert und im Zellüberstand spezifische ekto- oder entodermale Antigene immunologisch nachgewiesen werden, ohne daß auf den Zellüberstand abbauend eingewirkt wird.

2. Verfahren gemäß Anspruch 1, dadurch gekennzeichnet, daß die Zellen aus Knochenmark entstammen.

3. Verfahren gemäß einem der Ansprüche 1 oder 2, dadurch gekennzeichnet, daß die Zellen fraktioniert lysiert werden.

4. Verfahren gemäß Anspruch 4, dadurch gekennzeichnet, daß durch Zugabe von Ammoniumchlorid zunächst die Erythrozyten lysiert, die verbleibenden intakten Zellen abgetrennt und danach durch Ultraschall lysiert werden.

5. Verfahren gemäß einem der Ansprüche 1 bis 4, dadurch gekennzeichnet, daß der immunologische Nachweis der spezifischen ekto- oder entodermalen Antigene mittels eines ELISA-Tests durchgeführt wird.

6. Verfahren gemäß einem der Ansprüche 1 bis 5, dadurch gekennzeichnet, daß die spezifischen ekto- oder entodermalen Antigene Cytokeratine oder Fragmente hiervon sind.

7. Verfahren gemäß Anspruch 6, dadurch gekennzeichnet, daß Cytokeratin 19 oder Fragmente hiervon bestimmt werden.

8. Verwendung eines Testkits zum Nachweis von Mikrometastasen ekto- oder entodermaler Tumoren in einem Verfahren gemäß Anspruch 1, das in mindestens zwei getrennten Verpackungen ein Reagenz zur Zellyse und ein Reagenz zur Durchführung des immunologischen Nachweises der spezifischen ekto- oder entodermalen Antigene enthält.

9. Verwendung eines Testkits gemäß Anspruch 8, dadurch gekennzeichnet, daß in der einen Verpackung als Zellysereagenz Ammoniumchlorid und in der anderen Verpackung die Rezeptoren R1 und R2 sowie eine Festphase, an die der Rezeptor R1 gebunden ist oder gebunden werden kann, enthalten sind.

## Claims

1. Method for the detection of micrometastases of ectodermal or entodermal tumours, wherein cells from mesenchymal tissue are lysed and specific ectodermal or entodermal antigens are detected immunologically in the cell supernatant without the cell supernatant being degraded.

2. Method as claimed in claim 1, wherein the cells are derived from bone marrow.

3. Method as claimed in one of the claims 1 or 2, wherein a fractional lysis of the cells is carried out.

4. Method as claimed in claim 3, wherein firstly erythrocytes are lysed by addition of ammonium chloride, the remaining intact cells are separated and afterwards lysed by ultrasound.

5. Method as claimed in one of the claims 1 to 4, wherein the immunological detection of the specific ectodermal or entodermal antigens is carried out by means of an ELISA test.

6. Method as claimed in one of the claims 1 to 5, wherein the specific ectodermal or entodermal antigens are cytokeratins or fragments thereof.

7. Method as claimed in claim 6, wherein cytokeratin 19 or fragments thereof are determined.

8. Use of a test kit for the detection of micrometastases of ectodermal or entodermal tumours in a method as claimed in claim 1 which contains a reagent for cell lysis and a reagent for carrying out the immunological detection of the specific ectodermal or entodermal antigens in at least two separate package units.

9. Use of a test kit as claimed in claim 8, wherein one of the packages contains ammonium chloride as the cell lysis reagent and the other package contains the receptors R1 and R2 as well as a solid phase to which the receptor R1 is bound or can be bound.

## Revendications

1. Procédé de détection de micrométastases de tumeurs ecto- ou endothermiques, caractérisé par le fait que l'on lyse des cellules issues de tissu mésenchymatique et que dans le surnageant cellulaire, on détecte par voie immunologique des antigènes ecto- ou endothermiques spécifiques, sans action destructrice sur le surnageant cellulaire.

2. Procédé selon la revendication 1, caractérisé par le fait que les cellules sont issues de la moëlle osseuse.

3. Procédé selon l'une quelconque des revendications 1 ou 2, caractérisé par le fait que les cellules sont lysées de façon fractionnée.

4. Procédé selon la revendication 4, caractérisé par le fait que par addition de chlorure d'ammonium, on effectue d'abord la lyse des érythrocytes, on sépare les cellules intactes restantes et ensuite, on les lyse par ultrasons.

5. Procédé selon l'une quelconque des revendications 1 à 4, caractérisé par le fait que la détection immunologique des antigènes ecto- ou endothermiques spécifiques est réalisée au moyen d'un test ELISA.

6. Procédé selon l'une quelconque des revendications 1 à 5, caractérisé par le fait que les antigènes ecto- ou endothermiques spécifiques sont de la cytokératine ou des fragments de celle-ci.

7. Procédé selon la revendication 6, caractérisé par le fait que l'on détermine la cytokératine 19 ou des fragments de celle-ci.

8. Utilisation d'un coffret d'analyse pour la détection de micrométastases de tumeurs ecto- ou endothermiques suivant un procédé selon la revendication 1, qui contient, dans au moins deux conditionnement séparés, un réactif destiné à la lyse des cellules et un réactif destiné à la mise en oeuvre de la détection immunologique des antigènes ecto- ou endothermiques spécifiques.

9. Utilisation d'un coffret d'analyse selon la revendication 8, caractérisée par le fait que l'un des conditionnements contient, en tant que réactif de lyse cellulaire, du chlorure d'ammonium, et l'autre conditionnement contient les récepteurs R1 et R2 ainsi qu'une phase solide à laquelle le récepteur R1 est lié ou peut être lié.
